# EUROPÄISCHE PATENTSCHRIFT

(11) **EP 0 686 627 B1**
(45) Veröffentlichungstag und Bekanntmachung des Hinweises auf die Patenterteilung: **19.08.1998**
(21) Anmeldenummer: 95106922.8
(22) Anmeldetag: 08.05.1995
(51) Int. Cl.: C07C 317/46, C07C 317/44, A61K 31/155

(54) **Perfluoralkyl-substituierte Benzoylguanidine, Verfahren zu ihrer Herstellung, ihre Verwendung als Medikament oder Diagnostikum sowie sie enthaltendes Medikament**
Substituted perfluoroalkylguanidines, process for their preparation, their use as medicine or diagnostic as well as medicines containing them
Benzoylguanidines perfluoroalkyl-substituées, procédé de leur préparation, leur usage comme médicament ou diagnostique et médicaments les contenant

(30) Priorität: 13.05.1994 DE 4417004
(43) Veröffentlichungstag der Anmeldung: 13.12.1995
(73) Patentinhaber: HOECHST AKTIENGESELLSCHAFT, 65929 Frankfurt am Main (DE)
(72) Erfinder: Kleemann, Heinz-Werner, Dr., D-65474 Bischofsheim (DE); Lang, Hans-Jochen, Dr., D-65719 Hofheim (DE); Schwark, Jan-Robert, Dr., D-65929 Frankfurt (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(56) Entgegenhaltungen:
- EP-A- 0 556 673
- EP-A- 0 556 674
- EP-A- 0 577 024
- EP-A- 0 602 522
- EP-A- 0 627 413

## Beschreibung

Die Erfindung betrifft Benzoylguanidine der Formel I 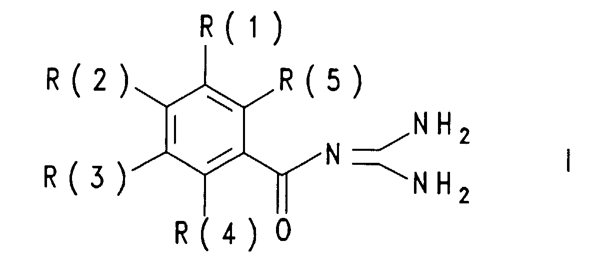 worin bedeuten:
- R(1): R(6)-SOₘ;
m Null, 1 oder 2;
R(6) Perfluoralkyl mit 1, 2, 3, 4, 5 oder 6 C-Atomen, das geradkettig oder verzweigt ist;
- R(2) und R(3): unabhängig voneinander Wasserstoff, F, Cl, Br, I, Alkyl mit 1, 2, 3 oder 4 C-Atomen, Alkoxy mit 1, 2, 3 oder 4 C-Atomen, Phenoxy,
das unsubstituiert ist oder substituiert ist mit 1 - 3 Substituenten ausgewählt aus der Gruppe bestehend aus F, Cl, Methyl und Methoxy;
oder
- R(2) und R(3): unabhängig voneinander Pyrrol-1-yl, Pyrrol-2-yl- oder Pyrrol-3-yl,
welches nicht substituiert ist oder substituiert mit 1 bis 4 Substituenten, ausgewählt aus der Gruppe bestehend aus F, Cl, Br, I, CN, Alkanoyl mit 2, 3, 4, 5, 6, 7 oder 8 C-Atomen, Alkoxycarbonyl mit 2, 3, 4, 5, 6, 7 oder 8 C-Atomen, Formyl, Carboxy, CF₃, Methyl, Methoxy;
- R(4) und R(5): unabhängig voneinander Wasserstoff, Alkyl mit 1, 2 oder 3-C-Atomen, F, Cl, Br, I, CN, OR(7), NR(8)R(9), -(CH₂)ₙ-(CF₂)ₒ-CF₃;
R(7), R(8) und R(9) unabhängig voneinander Wasserstoff oder Alkyl mit 1, 2, 3 oder 4 C-Atomen;
n Null oder 1;
o Null, 1 oder 2;
sowie deren pharmakologisch akzeptable Salze.

Bevorzugt sind Verbindungen der Formel I, in denen bedeuten:
- R(1): R(6)-SOₘ;
m Null, 1 oder 2;
R(6) Perfluoralkyl mit 1, 2, 3 oder 4 C-Atomen, das geradkettig oder verzweigt ist;
- R(2) und R(3): unabhängig voneinander Wasserstoff, F, Cl, Br, Alkyl mit 1, 2, 3 oder 4 C-Atomen, Alkoxy mit 1, 2, 3 oder 4 C-Atomen, Phenoxy,
das unsubstituiert ist oder substituiert ist mit 1 - 2 Substituenten ausgewählt aus der Gruppe bestehend aus F, Cl, Methyl und Methoxy;
oder
- R(2) und R(3): unabhängig voneinander Pyrrol-1-yl, Pyrrol-2-yl- oder Pyrrol-3-yl,
welches nicht substituiert ist oder substituiert mit 1 bis 2 Substituenten, ausgewählt aus der Gruppe bestehend aus F, Cl, Br, I, CN, Alkanoyl mit 2, 3, 4 oder 5 C-Atomen, Alkoxycarbonyl mit mit 2, 3, 4 oder 5 C-Atomen, Formyl, Carboxy, CF₃ oder Methyl;
- R(4) und R(5): unabhängig voneinander Wasserstoff, Alkyl mit 1, 2 oder 3-C-Atomen, F, Cl, Br, OH, NH₂, -(CF₂)ₒ-CF₃;
o Null, 1 oder 2;
sowie deren pharmakologisch akzeptable Salze.

Besonders bevorzugt sind Verbindungen der Formel I, in denen bedeuten:
- R(1): -SO₂CF₃;
- R(2) und R(3): unabhängig voneinander Wasserstoff, F, Cl, Br, Alkyl mit 1, 2, 3 oder 4 C-Atomen, Methoxy, Phenoxy,
das unsubstituiert ist oder substituiert ist mit einem Substituenten aus der Gruppe bestehend aus F, Cl, Methyl und Methoxy;
oder
- R(2) und R(3): unabhängig voneinander Pyrrol-1-yl, Pyrrol-2-yl- oder Pyrrol-3-yl,
welches nicht substituiert ist oder substituiert mit 1 bis 2 Substituenten, ausgewählt aus der Gruppe bestehend aus F, Cl, Br, I, CN, Alkanoyl mit 2, 3, 4 oder 5 C-Atomen, Alkoxycarbonyl mit 2, 3, 4 oder 5 C-Atomen, Formyl, Carboxy, CF₃ oder Methyl;
- R(4) und R(5): unabhängig voneinander Wasserstoff, Methyl, F, Cl, Br, OH, NH₂, -CF₃;
sowie deren pharmakologisch akzeptable Salze.

Enthält einer der Substituenten R(1) bis R(6) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können sowohl geradkettig wie verzweigt vorliegen.

Die Erfindung betrifft weiterhin ein Verfahren zur Herstellung einer Verbindung I, dadurch gekennzeichnet, daß man
eine Verbindung der Formel II 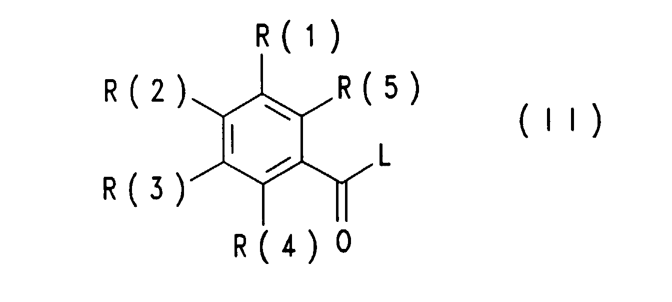 worin R(1) bis R(5) die angegebene Bedeutung besitzen und L für eine leicht nucleophil substituierbare leaving group steht,
mit Guanidin umsetzt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäurederivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol [L= = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit
O-[(Cyano(ethoxycarbonyl)methylen)amino]-1,1,3,3-tetramethyluroniumtetrafluorborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Die Einführung einiger Substituenten in 3-, 4- und 5-Stellung gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. -zink-Verbindungen.

Die Einführung einiger Substituenten in 4-Position gelingt durch literaturbekannte Methoden der nucleophilen Substitution am Aromaten.

Der Aufbau einer Perfluoralkylsulfonyl-Gruppe in 3-Position gelingt nach literaturbekannten Methoden aus dem Sulfonsäurefluorid mit Perfluoralkyltrimethylsilan.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.

Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 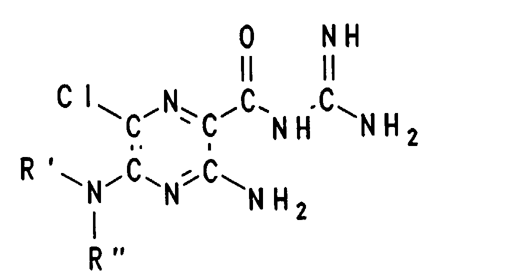 Amilorid: R',R" = H
Dimethylamilorid: R',R" = CH₃
Ethylisopropylamilorid: R' = C₂H₅, R'' = CH(CH₃)₂

Darüberhinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (suppl.1): 167 (1988) (book of abstracts)]. So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288) sind Benzoylguanidine beschrieben, die in der dem Rest R(1) entsprechenden Stellung ein Wasserstoff-Atom tragen. In der deutschen Patentanmeldung P 42 04 575.4 (HOE 92/F 034) werden Benzoylguanidine vorgeschlagen, in welchen aber die Substituenten nicht die nach der vorliegenden Erfindung beanspruchten Bedeutungen haben. Dieser Patentanmeldung entspricht das Südafrikanische Patent 93-0984.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, die für die Behandlung von Krankheiten wichtig sind, die beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺ /H⁺ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺ /H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht,Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 200 mg pro Tag notwendig werden.

Liste der Abkürzungen:

| | |
|---|---|
| MeOH | Methanol |
| DMF | N,N-Dimethylformamid |
| NBS | N-Bromsuccinimid |
| AIBN | α,α-Azo-bis-isobutyronitril |
| El | electron impact |
| DCI | Desorption-Chemical Ionisation |
| RT | Raumtemperatur |
| EE | Ethylacetat (EtOAc) |
| DIP | Diisopropylether |
| MTB | Methyltertiärbutylether |
| mp | Schmelzpunkt |
| HEP | n-Heptan |
| DME | Dimethoxyethan |
| FAB | Fast Atom Bombardment |
| CH₂Cl₂ | Dichlormethan |
| THF | Tetrahydrofuran |
| eq | Äquivalent |
| ES | Elektrospray-lonisation |

### Experimenteller Teil

Allgemeine Vorschrift zur Herstellung von Benzoyl-guanidinen (I)
Variante A: aus Benzoesäuren (II, L = OH)
0,01 M des Benzoesäurederivates der Formel II löst bzw. suspendiert man in 60 ml wasserfreiem THF und versetzt sodann mit 1,78 g (0,011 M) Carbonyldiimidazol. Nach dem Rühren über 2 Stunden bei RT werden 2,95 g (0,05 M) Guanidin in die Reaktionslösung eingetragen. Nach dem Rühren über Nacht destilliert man das THF unter vermindertem Druck (Rotavapor) ab, versetzt mit Wasser, stellt mit 2N HCI auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

Allgemeine Vorschrift zur Herstellung von Benzoyl-guanidinen (I)
Variante B: aus Benzoesäure-alkylestern (II, L = O-Alkyl)
5 mmol des Benzoesäure-alkylesters der Formel II sowie 25 mmol Guanidin (freie Base) werden in 15 ml Isopropanol gelöst oder in 15 ml THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotavapor) abdestilliert, in 300 ml EE aufgenommen und 3 x mit je 50 ml NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1

4-(4-Fluorphenoxy)-3-trifluormethylsulfonyl-benzoylguanidin 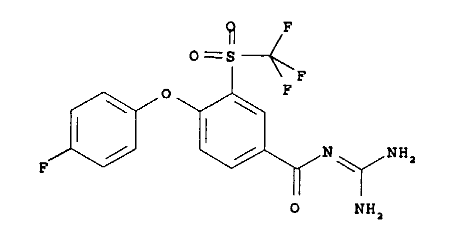
a) 2-Brom-5-methyl-benzolsulfonsäure
   140 g 2-Amino-5-methyl-benzolsulfonsäure werden in 810 g 30% wäßriger HBr-Lösung gelöst und bei 0 - 5°C eine Lösung von 51.8 g NaNO₂ in 100 ml Wasser zugetropft. 10 min wird bei 0°C nachgerührt und dann das Diazoniumsalz auf eine Lösung von 143.5 g CuBr in 250 ml halbkonzentrierter wäßriger HBr-Lösung gegossen. Anschließend wird auf dem Dampfbad vorsichtig erwärmt (ca 40 - 50°C), bis die Stickstoffentwicklung beendet ist. Es wird etwas Aktivkohle zugesetzt und heiß abfiltriert. Dann wird mit Wasser verdünnt und der Feststoff abfiltriert. Das Filtrat wird mit NaCl gesättigt, dabei fällt das gewünschte Produkt in Form farbloser Kristalle aus und wird abfiltriert. mp > 250°C.
   R_{f} (EE/MeOH 3:1) = 0.23 MS (DCI) : 252 (M + H)⁺
b) 2-Brom-5-methyl-benzolsulfonsäurechlorid
   50 g 2-Brom-5-methyl-benzolsulfonsäure und 41.5 g PCl₅ werden in 500 ml Toluol vorsichtig bis zum Einsetzen der Gasentwicklung erwärmt. Nach Abklingen der Reaktion wird unter Rückfluß gekocht, bis die Gasentwicklung beendet ist(2 h). Man läßt abkühlen und entfernt das Toluol und das POCl₃ im Vakuum. Der Rückstand wird vorsichtig auf etwa 1 l Eis gegeben, es wird 1 h nachgerührt und dann das Produkt abgesaugt. Trocknung bei 60°C im Vakuum liefert 36 g eines farblosen Feststoffs.
   MS (DCI) : 269 (M + H)⁺
c) 2-Brom-5-methyl-benzolsulfonsäurefluorid
   32 g 2-Brom-5-methyl-benzolsulfonsäurechlorid werden in 75 ml Dioxan gelöst und mit einer Lösung von 20.6 g KF in 20 ml Wasser versetzt. Man rührt 48 h bei 45°C, gießt auf 500 ml Wasser, rührt 30 min nach und saugt schließlich das Produkt ab. 24 g farbloser Feststoff.
   MS (DCI) : 253 (M + H)⁺
d) (2-Brom-5-methyl-phenyl)-trifluormethylsulfon
   21.4 g 2-Brom-5-methyl-benzolsulfonsäurefluorid werden in 225 ml einer 0.5 M Lösung von Me₃SiCF₃ in THF gelöst. Bei 25 - 30°C wird dann eine Lösung von 2.5 g Tris-(dimethylamino)-schwefel-(trimethylsilyl)-difluorid in 100 ml THF langsam zugetropft. 3 h wird bei RT gerührt, 200 ml gesättigte wäßrige NaCl-Lösung zugegeben und 3 x mit je 300 ml EE extrahiert. Über Na₂SO₄ wird getrocknet und das Solvens im Vakuum entfernt. Das Rohprodukt wird einmal mit EE/HEP 1 : 4 und ein zweites Mal mit MTB chromatographiert. Man erhält 13 g farbloser Kristalle, mp 69°C.
   R_{f} (EE/Hep 1:4) = 0.30 MS (DCI) : 303 (M + H)⁺
e) (2-Brom-5-tribrommethyl-phenyl)-trifluormethylsulfon
   1 g (2-Brom-5-methyl-phenyl)-trifluormethylsulfon, 350 µl Brom und eine katalytische Menge (ca 50 mg) Benzoylperoxid werden in 30 ml Chlorbenzol 8 h bei 130°C unter Bestrahlung mit einer 300 W Glühlampe gerührt. Stündlich wird eine neue katalytische Menge Benzoylperoxid zugegeben. Erneut werden 300 µl Brom zugegeben und weitere 14 h bei 130°C gerührt. Man läßt das Reaktionsgemisch abkühlen, mit überschüssiger wäßriger Na₂SO₃-Lösung wird entfärbt, 100 ml gesättigte wäßrige NaCl-Lösung zugesetzt und 3 x mit je 100 ml EE extrahiert. Über Na₂SO₄ wird getrocknet und das Solvens im Vakuum entfernt. Chromatographie mit EE/HEP 1 : 4 liefert 1.1 g eines farblosen Öls.
   R_{f} (EE/Hep 1:4) = 0.43 MS (DCI) : 537 (M + H)⁺
f) 4-Brom-3-trifluormethylsulfonyl-benzoesäuremethylester
   250 mg (2-Brom-5-tribrommethyl-phenyl)-trifluormethylsulfon werden in 3 ml MeOH gelöst und mit einer Lösung von 236 mg AgNO₃ in 5 ml Wasser versetzt. 30 min wird bei RT gerührt, der Niederschlag abfiltriert, das Filtrat mit überschüssiger wäßriger HCI-Lösung versetzt und erneut abfiltriert. Das Filtrat wird im Vakuum eingeengt und man erhält 190 mg eines blaßgelben Öls.
   R_{f} (DIP) = 0.59 MS (DCI) : 347 (M + H)⁺
g) 4-(4-Fluorphenoxy)-3-trifluormethylsulfonyl-benzoesäuremethylester
   500 mg 4-Brom-3-trifluormethylsulfonyl-benzoesäuremethylester, 162 mg 4-Fluorphenol und 600 mg K₂CO₃ werden in 10 ml DMF 1.5 h bei 120°C gerührt. Anschließend werden 100 ml Wasser zugegeben und 3 x mit je 100 ml EE extrahiert. Über Na₂SO₄ wird getrocknet und das Solvens im Vakuum entfernt. Chromatographie mit DIP liefert 278 mg eines farblosen Öls.
   R_{f} (EE/Hep 1:4) = 0.17 MS (DCI) : 379 (M + H)⁺
h) 4-(4-Fluorphenoxy)-3-trifluormethylsulfonyl-benzoylguanidin
   260 mg 4-(4-Fluorphenoxy)-3-trifluormethylsulfonyl-benzoesäuremethylester werden nach Variante B zu 53 mg Produkt umgesetzt, mp (Hydrochlorid) 160°C (Zersetzung).
   R_{f} (EE) = 0.32 MS (DCI) : 406 (M + H)⁺

### Beispiel 2

4-Isopropyl-3-trifluormethylsulfonyl-benzoylguanidin 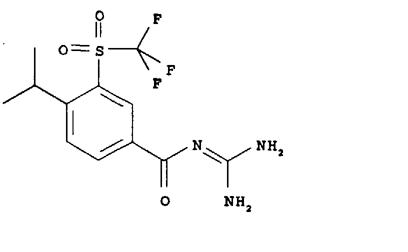
a) 2-Isopropyl-benzolsulfonsäurechlorid
   20 g 2-Isopropylthiophenol werden in 500 ml Wasser emulgiert. Bei etwa 10°C wird Chlor bis zur Sättigung eingeleitet und 30 min bei 10 - 20°C nachgerührt. Überschüssiges Chlor wird mit Stickstoff ausgeblasen und 3 x mit je 200 ml EE extrahiert. Über Na₂SO₄ wird getrocknet und das Solvens im Vakuum entfernt. Man erhält 27.0 g eines farblosen Öls.
   MS (DCI) : 219 (M + H)⁺
b) 2-Isopropyl-benzolsulfonsäurefluorid
   27 g 2-Isopropyl-benzolsulfonsäurechlorid und 22 g KF werden in 75 ml Dioxan und 20 ml Wasser 27 h bei 45°C und 45 h bei RT gerührt. Anschließend werden 800 ml Wasser zugegeben, und es wird 3 x mit je 300 ml EE extrahiert. Über Na₂SO₄ wird getrocknet und das Solvens im Vakuum entfernt. Man erhält 24 g eines farblosen Öls.
   MS (DCI) : 203 (M + H)⁺
c) (2-Isopropyl-phenyl)-trifluormethylsulfon
   17.4 g 2-Isopropyl-benzolsulfonsäurefluorid werden in 230 ml einer 0.5 M Lösung von Me₃SiCF₃ in THF gelöst. Bei 20-30°C werden dann 2.7 g Tris-(dimethylamino)-schwefel-(trimethylsilyl)-difluorid portionsweise zugegeben. 2 h wird bei RT gerührt und anschließend das Solvens im Vakuum entfernt. Der Rückstand wird in 500 ml EE aufgenommen und 2 x mit je 150 ml gesättigter wäßriger NaCl-Lösung gewaschen. Über Na₂SO₄ wird getrocknet und das Solvens im Vakuum entfernt. Man erhält 19.8 g eines farblosen Öls.
   R_{f} (EE/Hep 1:4) = 0.20 MS (DCI) : 253 (M + H)⁺
d) (2-Isopropyl-5-iod-phenyl)-trifluormethylsulfon
   2.52 g (2-Isopropyl-phenyl)-trifluormethylsulfon werden in 10 ml Trifluormethansulfonsäure gelöst und bei 0°C 2.55 g N-Iodsuccinimid zugegeben. 3 h wird bei RT gerührt, anschließend auf 100 ml Wasser gegossen und 3 x mit 100 ml Diethylether extrahiert. Die organische Phase wird dann nacheinander mit 100 ml gesättigter wäßriger Na₂CO₃-Lösung, 100 ml gesättigter wäßriger Na₂SO₃-Lösung und nochmal mit 100 ml gesättigter wäßriger Na₂CO₃-Lösung gewaschen. Über Na₂SO₄ wird getrocknet, das Solvens im Vakuum entfernt und mit EE/Hep 1 : 8 chromatographiert. Man erhält 2.2 g eines farblosen Öls.
   R_{f} (EE/Hep 1:8) = 0.46 MS (DCI) : 379 (M + H)⁺
e) 4-Isopropyl-3-trifluormethylsulfonyl-benzoesäuremethylester
   1.2 g (2-Isopropyl-5-iod-phenyl)-trifluormethylsulfon, 1.5 ml Tri-n-butylamin, 16 mg Pd(II)acetat und 27 mg 1,3-Bis-(diphenylphosphin)propan werden in 3 ml n-Butanol sowie 6 ml DMF gelöst und unter CO-Gas 5 h bei 100°C gerührt. Die Solventien werden im Vakuum entfernt, der Rückstand wird in 100 ml EE aufgenommen, und dann wird je 1 x mit 100 ml gesättigter wäßriger Na₂CO₃-Lösung und 100 ml gesättigter wäßriger NaHSO₄-Lösung gewaschen. Über Na₂SO₄ wird getrocknet und das Solvens im Vakuum entfernt. Chromatographie des Rückstandes mit EE/Hep 1:8 liefert 560 mg eines farblosen Öls.
   R_{f} (EE/Hep 1 : 8) = 0.32 MS (DCI) : 353
   (M + H)⁺
f) 4-Isopropyl-3-trifluormethylsulfonyl-benzoylguanidin
   560 mg 4-Isopropyl-3-trifluormethylsulfonyl-benzoesäuremethylester werden nach Variante B in 140 mg Produkt, farblose Kristalle umgewandelt. mp (Hydrochlorid) 192°C.
   R_{f} (EE) = 0.44 MS (DCI) : 338 (M + H)⁺

### Pharmakologische Daten:

### Inhibition des Na⁺ /H⁺ -Exchangers von Kaninchenerythrocyten

Weiße Neuseeland-Kaninchen (Ivanovas) erhielten eine Standard-Diät mit 2 % Cholesterin für sechs Wochen, um den Na⁺ /H⁺ -Austausch zu aktivieren und so den Na⁺ -Influx in die Erythrocyten via Na⁺ /H⁺ -Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-lnflux zu bestimmen, wurden 100 µl jeder Blutprobe in jeweils 5 ml eines hyperosmolaren Salz-Sucrose-Mediums (mmol/l: 140 NaCl, 3 KCI, 150 Sucrose, 0,1 Ouabain, 20 Tris-hydroxymethylaminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-lnflux ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

### Inhibition des Na⁺/H⁺-Exchangers:

| Beispiel | IC₅₀ (µmol/l) |
|---|---|
| 1 | 0.2 |
| 2 | 0.07 |

## Patentansprüche

1. Benzoylguanidine der Formel I 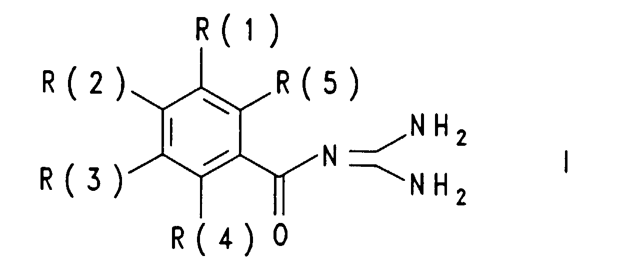 worin bedeuten:
R(1) R(6)-SOₘ;
m Null, 1 oder 2;
R(6) Perfluoralkyl mit 1, 2, 3, 4, 5 oder 6 C-Atomen, das geradkettig oder verzweigt ist;
R(2) und R(3) unabhängig voneinander Wasserstoff, F, Cl, Br, I, Alkyl mit 1, 2, 3 oder 4 C-Atomen, Alkoxy mit 1, 2, 3 oder 4 C-Atomen, Phenoxy,
das unsubstituiert ist oder substituiert ist mit 1 - 3 Substituenten ausgewählt aus der Gruppe bestehend aus F, Cl, Methyl und Methoxy;
oder
R(2) und R(3) unabhängig voneinander Pyrrol-1-yl, Pyrrol-2-yl- oder Pyrrol-3-yl,
welches nicht substituiert ist oder substituiert mit 1 bis 4 Substituenten, ausgewählt aus der Gruppe bestehend aus F, Cl, Br, I, CN, Alkanoyl mit 2, 3, 4, 5, 6, 7 oder 8 C-Atomen, Alkoxycarbonyl mit 2, 3, 4, 5, 6, 7 oder 8 C-Atomen, Formyl, Carboxy, CF₃, Methyl, Methoxy;
R(4) und R(5) unabhängig voneinander Wasserstoff, Alkyl mit 1, 2 oder 3-C-Atomen, F, Cl, Br, I, CN, OR(7), NR(8)R(9), -(CH₂)ₙ-(CF₂)ₒ-CF₃;
R(7), R(8) und R(9) unabhängig voneinander Wasserstoff oder Alkyl mit 1, 2, 3 oder 4 C-Atomen;
n Null oder 1;
o Null, 1 oder 2;
sowie deren pharmakologisch akzeptable Salze.

2. Verbindungen der Formel I nach Anspruch 1, in der bedeuten:
R(1) R(6)-SOₘ;
m Null, 1 oder 2;
R(6) Perfluoralkyl mit 1, 2, 3 oder 4 C-Atomen, das geradkettig oder verzweigt ist;
R(2) und R(3) unabhängig voneinander Wasserstoff, F, Cl, Br, Alkyl mit 1, 2, 3 oder 4 C-Atomen, Alkoxy mit 1, 2, 3 oder 4 C-Atomen, Phenoxy,
das unsubstituiert ist oder substituiert ist mit 1 - 2 Substituenten ausgewählt aus der Gruppe bestehend aus F, Cl, Methyl und Methoxy;
oder
R(2) und R(3) unabhängig voneinander Pyrrol-1-yl, Pyrrol-2-yl- oder Pyrrol-3-yl,
welches nicht substituiert ist oder substituiert mit 1 bis 2 Substituenten, ausgewählt aus der Gruppe bestehend aus F, Cl, Br, I, CN, Alkanoyl mit 2, 3, 4 oder 5 C-Atomen, Alkoxycarbonyl mit 2, 3, 4 oder 5 C-Atomen, Formyl, Carboxy, CF₃ oder Methyl;
R(4) und R(5) unabhängig voneinander Wasserstoff, Alkyl mit 1, 2 oder 3-C-Atomen, F, Cl, Br, OH, NH₂, -(CF₂)ₒ-CF₃;
o Null, 1 oder 2.

3. Verbindungen der Formel I nach einem der Ansprüche 1 oder 2, in der bedeuten:
R(1) -SO₂CF₃;
R(2) und R(3) unabhängig voneinander Wasserstoff, F, Cl, Br, Alkyl mit 1, 2, 3 oder 4 C-Atomen, Methoxy, Phenoxy,
das unsubstituiert ist oder substituiert ist mit einem Substituenten aus der Gruppe bestehend aus F, Cl, Methyl und Methoxy;
oder
R(2) und R(3) unabhängig voneinander Pyrrol-1-yl, Pyrrol-2-yl- oder Pyrrol-3-yl,
welches nicht substituiert ist oder substituiert mit 1 bis 2 Substituenten, ausgewählt aus der Gruppe bestehend aus F, Cl, Br, I, CN, Alkanoyl mit 2, 3, 4 oder 5 C-Atomen, Alkoxycarbonyl mit mit 2, 3, 4 oder 5 C-Atomen, Formyl, Carboxy, CF₃ oder Methyl;
R(4) und R(5) unabhängig voneinander Wasserstoff, Methyl, F, Cl, Br, OH, NH₂, -CF₃.

4. Verfahren zur Herstellung einer Verbindung I nach Anspruch 1, dadurch gekennzeichnet, daß man
eine Verbindung der Formel II 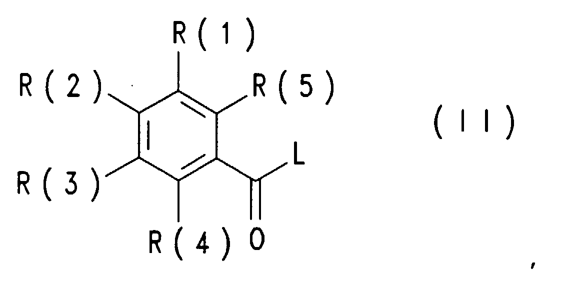 worin R(1) bis R(5) die angegebene Bedeutung besitzen und L für eine leicht nucleophil substituierbare leaving group steht, mit Guanidin umsetzt.

5. Verwendung einer Verbindung I nach Anspruch 1 zur Herstellung eines Medikaments zu Behandlung von Arrhythmien.

6. Verbindung l nach Anspruch 1 zur Anwendung bei der Behandlung von Arrhythmien, dadurch gekennzeichnet, daß man eine wirksame Menge einer Verbindung I nach Anspruch 1 mit den üblichen Zusatzstoffen versetzt und in einer geeigneten Darreichungsform verabreicht.

7. Verwendung einer Verbindung I nach Anspruch 1 zur Herstellung eines Medikaments zur Behandlung oder Prophylaxe des Herzinfarkts.

8. Verwendung einer Verbindung I nach Anspruch 1 zur Herstellung eines Medikaments zur Behandlung oder Prophylaxe der Angina Pectoris.

9. Verwendung einer Verbindung I nach Anspruch 1 zur Herstellung eines Medikaments zur Behandlung oder Prophylaxe von ischämischen Zuständen des Herzens.

10. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

11. Verwendung einer Verbindung I nach Anspruch 1 zur Herstellung eines Medikaments zur Behandlung oder Prophylaxe von ischämischen Zuständen peripherer Organe und Gliedmaßen.

12. Verwendung einer Verbindung I nach Anspruch 1 zur Herstellung eines Medikaments zur Behandlung von Schockzuständen.

13. Verwendung einer Verbindung I nach Anspruch 1 zur Herstellung eines Medikaments zum Einsatz bei chirurgischen Operationen und Organtransplantationen.

14. Verwendung einer Verbindung I nach Anspruch 1 zur Herstellung eines Medikaments zur Konservierung und Lagerung von Transplantaten für chirurgische Maßnahmen.

15. Verwendung einer Verbindung I nach Anspruch 1 zur Herstellung eines Medikaments zur Behandlung von Krankheiten, bei denen die Zellproliferation eine primäre oder sekundäre Ursache darstellt, und somit ihre Verwendung als Antiatherosklerotika, Mittel gegen Diabetische Spätkomplikationen, Krebserkrankungen, fibrotische Erkrankungen wie Lungenfibrose, Leberfibrose oder Nierenfibrose, Prostatahyperplasie.

16. Verwendung einer Verbindung I nach Anspruch 1 zur Herstellung eines wissenschaftliches Tools zur Inhibition des Na⁺ /H^{+ -}Exchangers, zur Diagnose der Hypertonie und proliferativer Erkrankungen.

17. Heilmittel, enthaltend eine wirksame Menge einer Verbindung I nach einem der Ansprüche 1 bis 3.

## Claims

1. A benzoylguanidine of the formula I 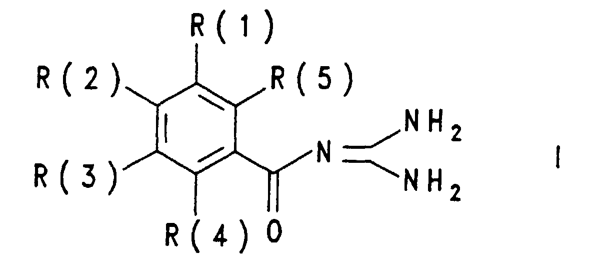 in which: -
R(1) is R(6)-SOₘ;
m is zero, 1 or 2;
R(6) is perfluoroalkyl having 1, 2, 3, 4, 5 or 6 carbon atoms which is straight-chain or branched;
R(2) and R(3) independently of one another are hydrogen, F, Cl, Br, I, alkyl having 1, 2, 3, or 4 carbon atoms, alkoxy having 1, 2, 3, or 4 carbon atoms, or phenoxy which is unsubstituted or is substituted by 1 - 3 substituents selected from the group consisting of F, Cl, methyl and methoxy;
or
R(2) and R(3) independently of one another are 1-pyrrolyl, 2-pyrrolyl or 3-pyrrolyl which is not substituted or is substituted by 1 to 4 substituents selected from the group consisting of F, Cl, Br, I, CN, alkanoyl having 2, 3, 4, 5, 6, 7 or 8 carbon atoms, alkoxycarbonyl having 2, 3, 4, 5, 6, 7 or 8 carbon atoms, formyl, carboxyl, CF₃, methyl and methoxy;
R(4) and R(5) independently of one another are hydrogen, alkyl having 1, 2, or 3 carbon atoms, F, Cl, Br, I, CN, OR (7) , NR(8)R(9), - (CH₂)ₙ- (CF₂)ₒ-CF₃;
R(7), R(8) and R(9) independently of one another are hydrogen or alkyl having 1, 2, 3 or 4 carbon atoms;
n is zero or 1;
o is zero, 1 or 2;
or a pharmacologically acceptable salt thereof.

2. A compound of the formula I as claimed in claim 1, in which:
R(1) is R(6)-SOₘ;
m is zero, 1 or 2;
R(6) is perfluoroalkyl having 1, 2, 3 or 4 carbon atoms which is straight-chain or branched;
or
R(2) and R(3) independently of one another are hydrogen, F, Cl, Br, alkyl having 1, 2, 3 or 4 carbon atoms, alkoxy having 1, 2, 3 or 4 carbon atoms, or phenoxy which is unsubstituted or is substituted by 1 - 2 substituents selected from the group consisting of F, Cl, methyl and methoxy;
or
R(2) and R(3) independently of one another are 1-pyrrolyl, 2-pyrrolyl or 3-pyrrolyl which is not substituted or is substituted by 1 to 2 substituents selected from the group consisting of F, Cl, Br, I, CN, alkanoyl having 2, 3, 4 or 5 carbon atoms, alkoxycarbonyl having 2, 3, 4 or 5 carbon atoms, formyl, carboxyl, CF₃ or methyl;
R(4) and R(5) independently of one another are hydrogen, alkyl having 1, 2 or 3 carbon atoms, F, Cl, Br, OH, NH₂, -(CF₂)ₒ-CF₃;
o is zero, 1 or 2.

3. A compound of the formula I as claimed in either of claims 1 or 2, in which:
R(1) is -SO₂CF₃;
R(2) and R(3) independently of one another are hydrogen, F, Cl, Br, alkyl having 1, 2, 3 or 4 carbon atoms, methoxy, or phenoxy which is unsubstituted or is substituted by one substituent from the group consisting of F, Cl, methyl and methoxy;
or
R(2) and R(3) independently of one another are 1-pyrrolyl, 2-pyrrolyl or 3-pyrrolyl which is not substituted or is substituted by 1 to 2 substituents selected from the group consisting of F, Cl, Br, I, CN, alkanoyl having 2, 3, 4 or 5 carbon atoms, alkoxycarbonyl having 2, 3, 4 or 5 carbon atoms, formyl, carboxyl, CF₃ or methyl;
R(4) and R(5) independently of one another are hydrogen, methyl, F, Cl, Br, OH, NH₂, -CF₃.

4. A process for the preparation of a compound I as claimed in claim 1, which comprises
reacting a compound of the formula II 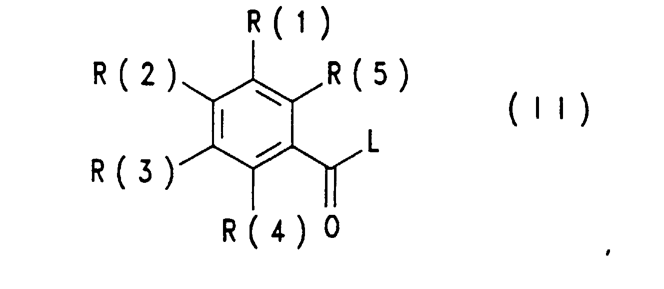 in which R(1) to R(5) are as defined and L is a leaving group which readily undergoes nucleophilic substitution, with guanidine.

5. The use of a compound I as claimed in claim 1 for the preparation of a medicament for the treatment of arrhythmias.

6. A compound I as claimed in claim 1 for use for the treatment of arrhythmias, wherein the conventional additives are added to an effective amount of a compound I as claimed in claim 1 and the composition is administered in a suitable dosage form.

7. The use of a compound I as claimed in claim 1 for the preparation of a medicament for the treatment or prophylaxis of cardiac infarction.

8. The use of a compound I as claimed in claim 1 for the preparation of a medicament for the treatment or prophylaxis of angina pectoris.

9. The use of a compound I as claimed in claim 1 for the preparation of a medicament for the treatment or prophylaxis of ischemic conditions of the heart.

10. The use of a compound I as claimed in claim 1 for the preparation of a medicament for the treatment or prophylaxis of ischemic conditions of the peripheral and central nervous system and of stroke.

11. The use of a compound I as claimed in claim 1 for the preparation of a medicament for the treatment or prophylaxis of ischemic conditions of peripheral organs and limbs.

12. The use of a compound I as claimed in claim 1 for the preparation of a medicament for the treatment of states of shock.

13. The use of a compound I as claimed in claim 1 for the preparation of a medicament for use in surgical interventions and organ transplants.

14. The use of a compound I as claimed in claim 1 for the preparation of a medicament for the preservation and storage of transplants for surgical interventions.

15. The use of a compound I as claimed in claim 1 for the preparation of a medicament for the treatment of diseases in which cell proliferation represents a primary or secondary cause, and thus their use as antiatherosclerotic agents, agents against diabetic late complications, cancerous diseases, fibrotic diseases such as pulmonary fibrosis, hepatic fibrosis or renal fibrosis, and prostate hyperplasia.

16. The use of a compound I as claimed in claim 1 for the preparation of a scientific tool for the inhibition of the Na⁺/H⁺ exchanger, for the diagnosis of hypertension and proliferative diseases. .

17. A medicament comprising an effective amount of a compound I as claimed in any one of claims 1 to 3.

## Revendications

1. Benzoylguanidines de formule I 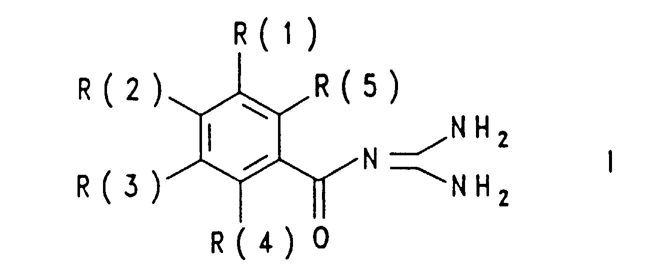 dans laquelle
R(1) représente R(6)-SOₘ,
m étant zéro, 1 ou 2,
R(6) étant un groupe perfluoroalkyle ayant 1, 2, 3, 4, 5 ou 6 atomes de carbone, qui est à chaîne droite ou ramifié;
R(2) et R(3) représentent, indépendamment l'un de l'autre, un atome d'hydrogène ou de F, CI, Br, I, ou un groupe alkyle à 1, 2, 3 ou 4 atomes de carbone, un groupe alcoxy à 1, 2, 3 ou 4 atomes de carbone, ou un groupe phénoxy
qui est non substitué ou est substitué par 1-3 substituants choisis dans l'ensemble constitué par F, CI, les groupes méthyle et méthoxy;
ou
R(2) et R(3) représentent, indépendamment l'un de l'autre, un groupe pyrrole-1-yle, pyrrole-2-yle ou pyrrole-3-yle,
qui est non substitué ou est substitué par 1 à 4 substituants choisis dans l'ensemble constitué par F, CI, Br, I, CN et les groupes alcanoyle à 2, 3, 4, 5, 6, 7 ou 8 atomes de carbone, alcoxycarbonyle à 2, 3, 4, 5, 6, 7 ou 8 atomes de carbone, formyle, carboxyle, CF₃, méthyle, méthoxy;
R(4) et R(5) représentent, indépendamment l'un de l'autre, un atome d'hydrogène ou un groupe alkyle à 1, 2 ou 3 atomes de carbone, F, CI, Br, I, CN, OR(7), NR(8)R(9), (CH₂)ₙ-(CF₂)ₒ-CF₃;
R(7), R(8) et R(9) représentant, indépendamment les uns des autres, un atome d'hydrogène ou un groupe alkyle à 1, 2, 3 ou 4 atomes de carbone,
n étant zéro ou 1,
o étant zéro, 1 ou 2;
ainsi que leurs sels pharmacologiquement acceptables.

2. Composés de formule I selon la revendication 1, dans lesquels
R(1) oreprésente R(6)-SOₘ,
m étant zéro, 1 ou 2,
R(6) étant un groupe perfluoroalkyle à 1, 2, 3 ou 4 atomes de carbone, qui est à chaîne droite ou ramifié;
R(2) et R(3) représentent, indépendamment l'un de l'autre, un atome d'hydrogène ou de F, CI, Br, ou un groupe alkyle à 1, 2, 3 ou 4 atomes de carbone, un groupe alcoxy à 1, 2, 3 ou 4 atomes de carbone, ou un groupe phénoxy
qui est non substitué ou est substitué par 1-2 substituants choisis dans l'ensemble constitué par F, CI, les groupes méthyle et méthoxy;
ou
R(2) et R(3) représentent, indépendamment l'un de l'autre, un groupe pyrrole-1-yle, pyrrole-2-yle ou pyrrole-3-yle, qui est non substitué ou est substitué par 1 ou 2 substituants choisis dans l'ensemble constitué par F, CI, Br, I, CN et les groupes alcanoyle à 2, 3, 4 ou 5 atomes de carbone, alcoxycarbonyle à 2, 3, 4 ou 5 atomes de carbone, formyle, carboxyle, CF₃ ou méthyle;
R(4) et R(5) représentent, indépendamment l'un de l'autre, un atome d'hydrogène ou un groupe alkyle à 1, 2 ou 3 atomes de carbone, F, CI, Br, OH, NH₂, -(CF₂)ₒ-CF₃;
o étant zéro, 1 ou 2.

3. Composés de formule I selon la revendication 1 ou 2, dans lesquels
R(1) représente -SO₂CF₃,
R(2) et R(3) représentent, indépendamment l'un de l'autre, un atome d'hydrogène ou de F, CI, Br, ou un groupe alkyle à 1, 2, 3 ou 4 atomes de carbone, le groupe méthoxy ou un groupe phénoxy qui est non substitué ou est substitué par un substituant choisi dans l'ensemble constitué par F, CI, les groupes méthyle et méthoxy;
ou
R(2) et R(3) représentent, indépendamment l'un de l'autre, un groupe pyrrole-1-yle, pyrrole-2-yle ou pyrrole-3-yle,
qui est non substitué ou est substitué par 1 ou 2 substituants choisis dans l'ensemble constitué par F, CI, Br, I, CN et les groupes alcanoyle à 2, 3, 4 ou 5 atomes de carbone, alcoxycarbonyle à 2, 3, 4 ou 5 atomes de carbone, formyle, carboxyle, CF₃ ou méthyle;
R(4) et R(5) représentent, indépendamment l'un de l'autre, un atome d'hydrogène ou le groupe méthyle, F, CI, Br, OH, NH₂, -CF₃;

4. Procédé pour la préparation d'un composé I selon la revendication 1, caractérisé en ce que l'on fait réagir avec de la guanidine un composé de formule II 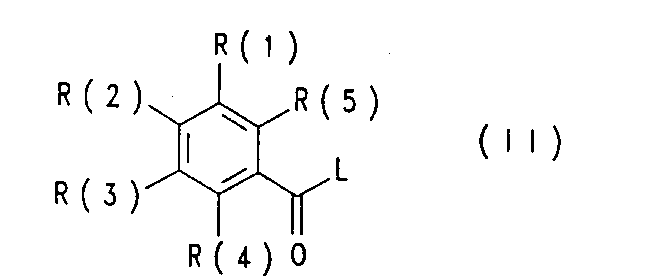 dans laquelle R(1) à R(5) ont les significations données plus haut et L représente un groupe partant pouvant être aisément remplacé par substitution nucléophile.

5. Utilisation d'un composé I selon la revendication 1, pour la fabrication d'un médicament destiné au traitement d'arythmies.

6. Composé selon la revendication 1, pour utilisation dans le traitement d'arythmies, caractérisé en ce que l'on ajoute les additifs usuels à une quantité efficace d'un composé I selon la revendication 1 et on l'administre sous une forme galénique appropriée.

7. Utilisation d'un composé I selon la revendication 1, pour la fabrication d'un médicament destiné au traitement ou à la prévention de l'infarctus du myocarde.

8. Utilisation d'un composé I selon la revendication 1, pour la fabrication d'un médicament destiné au traitement ou à la prévention de l'angine de poitrine.

9. Utilisation d'un composé I selon la revendication 1, pour la fabrication d'un médicament destiné au traitement ou à la prévention d'états ischémiques du coeur.

10. Utilisation d'un composé I selon la revendication 1, pour la fabrication d'un médicament destiné au traitement ou à la prévention d'états ischémiques du système nerveux périphérique et central et de l'apoplexie cérébrale.

11. Utilisation d'un composé I selon la revendication 1, pour la fabrication d'un médicament destiné au traitement ou à la prévention d'états ischémiques d'organes périphériques et des membres.

12. Utilisation d'un composé I selon la revendication 1, pour la fabrication d'un médicament destiné au traitement d'états de choc.

13. Utilisation d'un composé I selon la revendication 1, pour la fabrication d'un médicament destiné à l'utilisation dans des opérations chirurgicales et des greffes d'organes.

14. Utilisation d'un composé I selon la revendication 1, pour la fabrication d'un médicament destiné à la conservation et au stockage de transplants pour des opérations chirurgicales.

15. Utilisation d'un composé I selon la revendication 1, pour la fabrication d'un médicament destiné au traitement de maladies dans lesquelles la prolifération cellulaire représente une cause primaire ou secondaire, et par conséquent son utilisation en tant qu'agents anti-athérosclérose, agents contre les complications tardives diabétiques, les maladies cancéreuses, les fibroses telles que la fibrose pulmonaire, la fibrose hépatique ou la fibrose rénale, l'hyperplasie de la prostate.

16. Utilisation d'un composé I selon la revendication 1, pour la fabrication d'un outil scientifique dans l'inhibition de l'échangeur de Na⁺/H⁺, dans le diagnostic de l'hypertonie et de maladies proliférantes.

17. Médicament caractérisé par une teneur efficace en un composé de formule I selon l'une des revendication 1 à 3.
